# EUROPEAN PATENT SPECIFICATION

(11) **EP 0 348 120 B1**
(45) Date of publication and mention of the grant of the patent: **17.08.1994**
(21) Application number: 89306149.9
(22) Date of filing: 16.06.1989
(51) Int. Cl.: A01N 63/00

(54) **Method for screening bacteria and application thereof for field control of the weed downy brome**
Verfahren zum Auswählen von Bakterien und deren Verwendung in der Feldbekämpfung des Unkrauts Dachtrespe
Méthode de sélection de bactéries et utilisation de celles-ci dans la lutte sur les lieux contre la mauvaise herbe brome des toits

(30) Priority: 16.06.1988 US 207592
(43) Date of publication of application: 27.12.1989
(73) Proprietor: THE UNITED STATES OF AMERICA as represented by the Secretary United States Department of Commerce, Springfield, Virginia 22161 (US)
(72) Inventor: Elliott, Lloyd F., Bakersfield California 93309 (US); Kennedy, Ann C., Pullman Washington 99163 (US)
(74) Representative: Lord, Hilton David

(56) References cited:
- EP-A- 0 106 504
- US-A- 4 647 533

## Description

### BACKGROUND OF THE INVENTION

### 1. Field of the Invention

The invention relates to the isolation, selection, and application of novel strains of bacteria which have the ability to control the weed downy brome in small grain crops without deleteriously affecting the crop.

### 2. Description of the Art

Downy brome (Bromus tectorum) and the closely related species Japanese brome (B. japonicus), and cheat (B. secalinus),hereinafter referred to collectively as downy brome, are winter annual grass weeds which cause serious reductions in small grain crop yields by competing with crops for moisture, space and nutrients. This results in significant economic losses. The spread of these weeds has increased in recent years because conservation tillage systems make these weeds more difficult to control than conventional tillage systems.

Downy brome is a major weed of winter crops especially winter wheat. It can also be a problem in winter barley but is of less economic importance because winter barley acreage is relatively small. Winter wheat yield losses can be as high as 45% with 84-100 downy brome weeds/yard² while losses of 10% occur with a reduced downy brome infestation (Stahlman, Kansas Agricultural Experiment Station 87-69-S, 1986). Downy brome control of greater than 80% with the herbicide dicloflop resulted in 129 to 263% increased grain yield when compared to the control (Thill et al. In; STEEP Conservation Concepts and Accomplishments, L. F. Elliott et al. (eds.), pages 275-287, Washington State University Press (1987)). However, no chemical herbicide presently available gives consistant control against this weed.

While use of antagonistic microorganisms for biological control of some weeds have been reported, no bacterial strain has been previously found which would control the weed downy brome; additionally, no procedure for the selection of bacteria which inhibit downy brome in small grain crops has been reported. Because the physiological characteristics required for a bacterial strain to control weeds are very specific as to (1) the weed to be controlled; (2) the mode of action of weed control; (3) the activity and ecological niche of the microorganism; and (4) cultural practices and soil and climatic conditions favorable for control, information about microorganism treatments for control of weeds other than downy brome cannot be used to predict strains of microorganisms which would reduce downy brome under field conditions or predict criteria for selecting such strains.

### SUMMARY OF THE INVENTION

We have discovered a novel method for screening bacteria for selection of those strains which will inhibit (reduce the incidence or severity of) the growth of the weed downy brome in small grain crops under field conditions and a practical and effective method for application of the inhibitory bacteria to suppress downy brome growth in the field.

The bacteria selected by our method inhibit the growth of downy brome in small grain crops such as wheat, barley, and related crops without deleteriously affecting the crops. When applied as a spray or on straw, the novel bacteria obtained our method have the ability to inhibit downy brome under field conditions.

By use of our method, we have also discovered three novel strains initially determined as non-fluorescent pseudomonads which are effective in inhibiting growth of downy brome in field grown wheat.

Our screening method comprises:
1. Isolating strains of bacteria having potential for inhibiting downy brome from (a) the rhizoplane (root surface) or rhizosphere (soil immediately surrounding the roots) or both rhizoplane and rhizosphere of downy brome plants or (b) the rhizoplane or rhizosphere or both rhizoplane and rhizosphere of the crop variety (or related crop variety) to be protected in the field.
2. Screening the bacterial strains isolated in step 1 in vitro by growing downy brome in agar in the presence of a cell culture, substantially cell-free culture supernatant, or cell-free culture filtrate of the isolate being tested; growing control downy brome in the same manner but without the addition of the cell culture, supernant, or filtrate, and selecting as inhibitory bacteria those strains which inhibit downy brome as shown by reduction in root growth or reduction in germination when compared to the control. Those strains which inhibit downy brome are then tested in vitro against the small grain crop of the variety to be protected by growing the small grain crop in agar in the presence of a cell culture, substantially cell-free culture supernatant, or cell-free culture filtrate of the isolate being tested; growing control small grain crop plants in the same manner but without the addition of the cell culture, supernatant, or filtrate, and selects those strains which do not deleteriously affect the growth of the small grain crop.
3. Subjecting the bacterial strains selected in step 2 to a first screening in soil by separately growing downy brome plants and the small grain crop of the variety to be protected in a growth chamber or greenhouse in the presence of the bacterial strain selected in step 2. The bacterial concentration used is that which maximizes the selection of the number of strains that have the potential to inhibit downy brome in the field without deleteriously affecting the crop to be protected from downy brome and minimizes the selection of field-ineffective strains; growing control downy brome and small grain crop plants in a similar manner but without the addition of the bacterial strain, and selecting as inhibitory bacteria those strains which inhibit downy brome as shown by reduction in root growth or reduction in shoot growth when compared to the control downy brome plants without deleteriously affecting the small crop to be protected.
4. Screening the bacterial strains selected in step 3 in the field by separately growing downy brome plants and the small grain crop to be protected in the field in the presence of the bacterial strain selected in step 3 in a concentration which maximizes the selection of the number of strains which have the potential to inhibit downy brome in the field without deleteriously affecting the crop to be protected from downy brome and minimizes the selection of field-ineffective strains; growing control downy brome and small grain crop plants in the same manner but without the addition of the bacteria, and selecting as field-inhibitory bacteria those strains that inhibit downy brome as shown by reduction in stand, reduction in root growth, or reduction in shoot growth when compared to the control plants without deleteriously affecting the small grain crop to be protected.

The bacterial strains selected by our method have the ability to inhibit downy brome in a commerical setting without deleteriously affecting the small grain crop to be protected when used as a treatment on the crop area to be protected. Such treatments include spraying an inhibitory amount of the selected bacteria or applying straw containing an inhibitory amount of the selected bacteria on the crop area after planting. Both methods are practical and economical for controlling downy brome growth in small grain crop stands. The former method is particularly suited to the crop growing in heavy residues, the latter to the crop growing in areas with little or no surface residues.

The need for a biological control of downy brome in cropland has long been recognized as crop losses due to this weed have been significant and control by herbicides impractical. By using the procedures and conditions of our screening method, strains of bacteria can be selected which will inhibit downy brome growth in the field. By using our application method, downy brome growth can be controlled in a commercial setting.

In accordance with this discovery, it is an object of the invention to provide a means for screening bacteria to select those strains which inhibit downy brome in small grain crops under field conditions without deleteriously affecting the small grain crop to be protected.

It is also an object of the invention to provide a method for biologically controlling downy brome in small grain crops under field conditions using the strains selected by our method.

Another object of the invention in the provision of a method to identify root colonizing non-fluorescent pseudomonads that inhibit downy brome and utilize the strong root colonizing ability of the organisms to establish on the roots of downy brome in the field. Similar to other bacterial strains, it in difficult to take these organisms from the growth chamber to the field due to variables such as soil conditions, soil moisture, temperature, and the liked. By the use of our method, however, it in possible to access bacteria for field effectiveness and to apply downy brome-inhibitory bacteria for control of downy brome in small grain crops.

Another object of the invention in the provision of novel strains of pseudomonads which inhibit downy brome on field grown wheat without deleteriously affecting the wheat.

Other objects and advantages of the invention will become apparent from the ensuing description.

### DESCRIPTION OF THE PREFERRED EMBODIMENTS

The screaming method comprises:

### Step 1. Isolation of Strains of Potentially Inhibitory Bacteria.

To biologically control downy brome, a bacterial strain must have the ability to establish and grow in the microhabitat where it is to be used to inhibit the growth of the weed. This means it must have the ability to colonize the root system of downy brome. For the purposes of thin invention, the term "downy brome" as used herein includes three closely related species of the genus Bromus, downy brome (Bromus tectorum), Japanese brome (B. japonicus), and cheat (B. secalinus). Such bacteria are selected by isolating strains from the rhizoplane (root surface) or rhizosphere (soil immediately surrounding the roots) or both rhizoplane and rhizosphere of (a) downy brome plants or (b) small grain crops. Although strains from the rhizoplane or rhizosphere of one species of downy brome or one small grain may be isolated in this step for potential use to control another species of downy brome or another small grain crop, it is preferred that the bacterial strain be isolated from the species of downy brome to be inhibited or from the variety of small grain to be protected as it is likely to best function in the ultimate biocontrol habitat.

It is within the compass of the invention to isolate any type of bacteria having the potential to inhibit downy brome without deleteriously affecting the small grain crop to be protected, however non-fluorescent pseudomonads are the bacteria of choice because (1) they can be easily isolated, cultured, and identified; (2) they normally inhabit rhizosphere or rhizoplane soil, (3) they are nutritionally versatile, being able to utilize a large number of organic substrates, including, root exudates, (4) some strains produce toxins that inhibit downy brome; and (5) they can be successfully introduced into the root system of small grain crops and become established in the rhizosphere and rhizoplane.

Winter wheat is the small grain crop which is most significantly affected by downy brome by reduction in crop yield and resultant economic loss. For the purposes of description of our method, winter wheat is used as exemplary of the crop to be protected, however, our method is applicable to other small grains crops, for example, barley, oats, rye, triticale, and related crops.

The isolation procedure is as follows: winter wheat plants or downy brome plants are dug from the field and adhering soil gently washed from the roots with flowing tapwater. The roots are excised from the tops. The bacteria are collected and isolated by standard procedures. For example, bacteria of the genus Pseudomonas can be isolated by placing the excised roots in sterile dilutions blanks containing sterile water and glass beads and shaking the mixture vigorously to remove organisms clinging to the root surface. Then serial dilutions are prepared and spread plated on to a Pseudomonas selective medium such as King's Medium B supplemented with novobiocin, penicillin, and cycloheximide (KMB NPC) (Sands and Rovira, Applied Microbiology 20:513-514 (1970)), and incubated at a suitable temperature, e.g., 20^{o}C. Individual non-fluorescent colonies are identified by viewing the plates under ultraviolet light and selecting those colonies that do not exhibit fluorescence. For short term storage during the screening procedure, inoculum from individual non-fluorescent colonies is placed on a KMB NPC slant.

### Step 2. Screening of the Bacterial Strains In Vitro.

Bacterial strains isolated in step 1 are screened to select those strains which inhibit downy brome growth in vitro as exhibited by reduction in root growth or germination as compared to control plants. Those strains which are inhibitory to downy brome are then tested against wheat; those strains which do not deleteriously affect wheat growth in vitro are selected.

In the in vitro screening procedure, individual non-fluorescent colonies are inoculated into a medium suitable for growing up the colonies, for example Pseudomonas minimal salts medium (PMS) described by Gasson, Applied and Environmental Microbiology 39: 25-29 (1980), and grown at at a suitable temperature, e.g., about 20^{o}C. until late logarithmic growth (about 10⁹ to 10¹¹ cells per ml culture medium). The cells are then treated in at least one of the following ways: (1) the culture is used directly, (2) the culture is centrifuged to obtain a substantially cell-free culture supernatant (not more than about 10⁴ cells per ml of supernatant), or (3) the cell culture is centrifuged and filter sterilized to obtain a cell-free culture filtrate (no cells present). The first method assays both the effect of the organism and the effect of toxin production by the organism on downy brome growth. The second method is a quick assay to determine the effect of toxin production by the organism on the growth of downy brome. The third method assays only the effect of toxin production by the organism on downy brome growth. We have found that the assays for toxin production appear to provide for better selection of effective strains.

Downy brome growth inhibition is assessed by adding to petri plates a standard quantity, usually 2 ml, of the cell culture, substantially cell-free culture supernatant, or cell-free culture filtrate. A standard quantity, usually 18 ml, of 0.9% molten agar (50^{o}C) is added to each plate, the contents mixed, and allowed to solidify. Generally two plates are prepared for each organism tested. Control plates are prepared by using PMS instead of the cell culture, substantially cell-free culture supernatant, or cell-free culture filtrate. The plates are planted with pregerminated downy brome seeds, 15 seeds per plate is convenient, and allowed to grow at a suitable temperature, e.g., 15^{o}C. Before root growth from the seedlings interferes with each other, about 3-6 days, the seedlings are pulled from the agar and root length or germination or both are recorded. The organisms showing significant inhibition of downy brome growth (length) or germination when compared to the control are tested against winter wheat seedling growth. For the purposes of this invention, significant inhibition of downy brome in the in vitro test is defined as at least a 50% reduction in root growth (length) when compared to the control or at least a 20% reduction in germination when compared to the control.

The strain selected above is assessed against wheat as follows: Test tubes receive a standard quantity, usually 1 ml each, of the cell culture, substantially cell-free culture supernatant, or the cell-free culture filtrate which was used in the downy brome test, and a standard quantity of 0.9% molten agar (50^{o}C), usually about 9 ml, is added to each tube, the contents mixed, and the tube slanted. Generally 10 growth tubes are prepared for each organism tested. Control tubes are prepared identically except that PMS is substituted for the cell culture, substantially cell-free culture supernatant, or the cell-free culture filtrate. When solidified, pregerminated winter wheat seeds (one per tube) are planted mid-slope on the slants and allowed to grow at a suitable temperature, e.g., 15^{o}C. Before root growth reaches the bottom of the tube, approximately 3-6 days, the seedlings are pulled from the growth tubes and root length recorded. A bacterial strain is denoted as one that does not deleteriously affect winter wheat when root growth reduction of the bacterial treated wheat is less than 10% when compared to the control wheat seedlings.

Pure cultures of the strains significantly inhibiting downy brome growth with little or no effect on winter wheat growth are individually streaked onto a suitable medium such as KMB NPC and selected and restreaked until the strain is pure and stable. Each individual strain is grown up and maintained so as to keep it stable such as by storing in glycerol at -10^{o}C or lyophilizing and storing at -10^{o}C.

### Step 3. Screening of the Bacterial Strains in the Growth Chamber.

Bacterial strains isolated in the step 2 are subjected to a first screening in soil by separately growing downy brome plants and wheat plants in a growth chamber or greenhouse in the presence of the bacterial strain selected in step 2. In this step, those strains are selected which, at a particular concentration, inhibit downy brome without deleteriously affecting wheat.

In this test, pots are filled with soil and seeded with downy brome or with wheat seeds. We have found that 6.4 cm diameter by 7.6 cm deep plastic pots seeded with 10 downy brome seeds and 7.6 cm diameter by 15.2 cm deep plastic pots seeded with four winter wheat seeds are convenient sizes for growth chamber or greenhouse studies. Any soil suitable for growth chamber studies can be used. One exemplary soil is Ritzville silt loam amended with 20% sand by weight. It is not necessary to surface-sterilize the seeds prior to planting.

The amount of bacteria per downy brome seed in the test pots is selected so as to optimize the selection of field-effective strains and minimize the selection of field-ineffective strains. For non-fluorescent pseudomonads, we have found that a range of 10⁸ to 10¹⁰ colony forming units (CFU) of the test strain per downy brome test pot is applied to the soil surface. One convenient method is putting the test strain in a liquid such as distilled water or PMS and dripping the liquid on to the soil surface. Wheat seeds are grown in the presence of 10⁸ to 10¹⁰ CFU of the test strain per pot with 4 seeds to assess the effect of the bacteria on wheat. Separate downy brome and winter wheat controls are prepared identical to the test samples except without bacterial treatment.

The soil is wetted to provide good plant growth, and the pots incubated in the growth chamber. We have found that a 14-hour day at 18^{o}C and a 10-hour night at 13^{o}C is suitable. After about 3 to 4 weeks, the downy brome and wheat seedlings are pulled up and the roots washed with water until free of soil. The roots and shoots of the downy brome and wheat seedlings are excised and roots or shoots or both dried to remove water so that a comparison to control plants can be made. Drying at 60^{o}C for 48 hours is suitable.

### Evaluation of Seedlings in the Growth Chamber Test:

To evaluate the bacterial treatment, root or shoot dry weight of the downy brome plants is compared with the root or shoot dry weight of the control downy brome plants. Bacterial strains which cause the treated downy brome seedlings to have reduced root growth (reduced root dry weight when compared to the control) of at least 25% or which cause the treated downy brome seedlings to have reduced short growth (reduced shoot dry weight when compared to the control) of at least 25% are considered inhibitory to downy brome in this test. While reduction in germination of downy brome compared with the control is not necessary, we have found that the additional criterion of selecting strains which showed a 20% or greater reduction in germination useful. To obtain germination values, emergence of downy brome grown in the presence of the test strain is counted and compared with the control plants.

To evaluate the bacterial treatment on wheat, root dry weight or shoort dry weight of the wheat plants are compared with the control wheat plants. For the purposes of this invention, a bacterial strain is denoted as one that does no deleteriously affect winter wheat when reduction in root growth (root dry weight) or shoot growth (shoot dry weight) of the bacterial treated wheat is less than 10% when compared to the control wheat seedlings.

### Step 4. Screening the Bacterial Strains in the Field

Bacterial strains selected in the previous step are next tested in the field. Treatment and control plots are laid out. We have found that treatment plots consisting of a meter² are suitable. The control (non-treatment) should be within close proximity to the comparable treatment plot so that treatment and non-treatment plots have similar soil conditions.

For the field test, it is preferred that downy brome plants be grown in plots that are substantially free of other weeds so that variability due to the presence of other weeds is reduced. This can be accomplished by chemical herbicide application or fumigation prior to planting. We have found it useful to have the previous crop residues of the small grain crop, e.g., barley or wheat, left on the soil surface as this provides a substrate for the inoculum. A convenient plot size is four 1-meter rows of downy brome per treatment with a control plot within about 6 meters of the treatment plot. The plots are seeded to downy brome, about 70 to 150 seeds per meter². Individual bacterial strains to be tested are applied to the soil as a spray treatment or on straw. The treatment is applied just after planting to up to 4 weeks. The spray treatment is economical and more convenient; straw provides protection for inoculum under adverse weather conditions. For the spray treatment, the test strain in a liquid such as distilled water or PMS is sprayed on to the soil to provide a concentration of about 10⁷ to 10¹² and preferably about 10⁸ CFU of the test strain per meter². The straw treatment is prepared by placing straw chopped into about 5 cm lengths into a tank and inoculating with the strain being tested. Water potential of the straw is adjusted to provide good growth of the organism. Thereafter, the straw is mixed about once a day. After about 5 days at about 15-20^{o}C., the bacteria ranges about 10⁹ to 10¹⁰ CFU of the test strain per gram of straw. Straw is added to the plots to give about 10⁷ to 10¹², and preferably about 10⁸ CFU of the test strain per meter². Control plots are treated identical to the test plot except that no bacteria are applied. To have statistical significance, each organism treatment is replicated a minimum of three times.

Plots seeded to winter wheat (about 100 to 150 seeds per meter²) are seeded beside the downy brome plots and the test organisms are sprayed on to the plots to provide a concentration of about 10⁷ to 10¹², and preferably about 10⁸ CFU of the test strain per meter². A convenient plot size is a meter². The treatment is applied just after planting to up to 4 weeks after planting. The control plants receive identical treatment except that no bacteria are applied. To have statistical significance, each organism treatment is replicated a minimum of three times.

### Evaluation of Seedlings in the Field Test:

To assess the effect of the bacterial treatment in the inhibition of downy brome in the field, after adequate growth has occurred, e.g., about the 5-leaf stage, the downy brome plants are harvested, roots washed, and at least one of the following is obtained: root dry weight, shoot dry weight, or stand (numbers of plant per meter length). Bacterial strains which cause the treated downy brome plants to average a reduction in root growth (root dry weight), reduction in shoot growth (shoot dry weight), or reduction in stand of at least 10% when compared to control downy brome plants are considered inhibitory to downy brome in the field test. To assess the effect of the bacterial treatment on winter wheat, it is harvested, roots washed, and at least one of the following is obtained: root dry weight, shoot dry weight, or stand (numbers of plant per meter length). Those bacterial strains which cause winter wheat to average less than 10% reduction in root growth (root dry weight), shoot growth (shoot dry weight) or stand compared to control wheat are defined as not deleteriously affecting the crop.

### Application of Downy Brome-Inhibitory Bacteria For Control of Downy Brome in a Commercial Setting

For convenience or economy of application, one of two methods is useful to control downy brome in a commercial setting depending on whether the field has surface residues or bare soil. The inhibitory organisms grow well in straw; if it is desired to control downy brome in wheat sown into previous crop residues on the soil surface, spraying the organisms on the residues may be the most convenient method of application. It is best to spray after a rain or shortly before and during periods of high rain probability. Where prolonged hot-dry periods may occur, application of inoculated straw or similar material may give better results. Application of the organisms in either case should be done after seeding up to 4 weeks after seeding.

Individual strains of bacteria which inhibited downy brome in the growth chamber and field tests without deleteriously affecting wheat are cultured by standard methods for a time to grow sufficient bacteria to treat the field area, generally about 48 hours for spray treatments or for about 5 days on straw or similar material for straw treatment. After wheat is seeded into the field, the organisms are applied to the field in an amount sufficient to inhibit downy brome. For non-fluorescent pseudomonads, this amount is about 10⁷ to 10¹² CFU of inhibitory bacteria per meter².

Statement of Deposit. Strains initially determined as non-fluorescent pseudomonads that passed the above screening test have been deposited in the Agricultural Research Culture Collection (NRRL) in Peoria, Illinois 61604 and have been assigned the following accession numbers: NRRL B-18293, NRRL B-18294, and NRRL B-18295.

### EXAMPLES

The following examples are intended only to further illustrate the invention and are not intended to limit the scope of the invention which is defined by the claims.

### EXAMPLE 1

### Isolation of Pseudomonas Strains.

Winter wheat and downy brome plants were dug from the field and the roots washed free from adhering soil in flowing tapwater. The roots were excised and placed in a sterile dilution blank containing 95 ml of deionized water and glass beads. The mixture was vigorously shaken on a wrist-action shaker and serial dilutions prepared and spread plated on King's Medium B supplemented with 45 mg novobiocin, 45 mg penicillin G, and 75 mg cyclohexamide (Sands and Rovira, supra). Non-fluorescent pseudomonads were identified by viewing the plates under ultraviolet light after 2-3 days of incubation at 20^{o}C.

### EXAMPLE 2

### Initial Isolate Screening

Individual non-fluorescent colonies obtained by the procedure outlined in Example 1 were screened in vitro as follows: Individual isolates were grown in PMS broth (Gasson, supra, KCl, 0.2 g; NH₄H₂PO₄, 1.00 g: H₂O, 100 ml; adjusted to pH 7.0, after autoclaving 20 ml sterile 2% MgSO₄.7H₂O, and 20 ml sterile 10% glucose added) at 20^{o}C until late logarithmic growth (0.9 O.D. at 500 nm on the spectrophotometer). The cultures were then used to inoculate the downy brome assay directly, centrifuged to obtain a substantially cell-free culture supernatant, or centrifuged and filter sterilized to obtain a cell-free culture filtrate.

Plastic petri dishes received 2 ml each of the cell culture, substantially cell-free culture supernatant, or the cell-free culture filtrate. Control plates received 2 ml of fresh PMS. Then 18 ml of 0.9% molten bacto-agar (Difco) (50^{o}C) were added to each plate, and the contents mixed. When solidified, 15 pregerminated downy brome seeds were planted on each plate and allowed to grow at 15^{o}C. Before root growth from the seedlings interfered with each other (5 days), the seedlings were pulled from the agar and root and shoot length were recorded. The organisms which showed at least a 50% reduction in root growth when compared to the control were denoted as inhibitory to downy brome and were tested against winter wheat seedling growth. Test tubes received 1 ml each of the cell culture, substantially cell-free culture supernatant, or the cell-free culture filtrate. Control tubes received 1 ml of fresh PMS. Then 9 ml of 0.9% molten bacto-agar (50^{o}C) were added to each tube, the contents mixed, and the tubes slanted. When solidified, a pregerminated wheat seed was planted mid-slope on each slant and allowed to grow at 15^{o}C. Before root growth reached the bottom of the tube (3 days), the seedlings were pulled from the growth tubes and root and shoot length recorded. In order for the bacterial strain to be considered not to be deleterious to winter wheat, the wheat seedlings treated with the bacteria must have averaged less than 10% reduced root growth when compared to the control plants.

Over 300 non-fluorescent Pseudomonas have been tested for inhibition of downy brome growth. Of these, about six strains have shown strong inhibition of downy brome growth with no effect on winter wheat growth. The test results for three strains which passed the in vitro screening test are given in Table 1. Cultures retained for further studies were individually streaked and selected and restreaked until the strain was pure and stable and stored in sterile aqueous glycerol at -10^{o}C.

**TABLE 1**

| Isolate | Downy Brome | | Winter Wheat | |
|---|---|---|---|---|
| | Root Length (mm) | Reduction In Root Growth Compared to Control (%) | Root Length (mm) | Reduction In Root Growth Compared to Control (%) |
| Control | 8.8 | -- | 33.2 | -- |
| NRRL B-18293 | 2.4 | 73 | 37.8 | None |
| NRRL B-18294 | 1.8 | 80 | 43.5 | None |
| NRRL B-18295 | 3.2 | 64 | 35.3 | None |

### EXAMPLE 3

### Screening of Bacterial Strains in the Growth Chamber.

Pseudomonas strains initially determined as non-fluorescent NRRL B-18294, and NRRL B-19295 that were inhibitory to downy brome but not deleterious to wheat as described in Example 2 were screened in the growth chamber to determine the effect of the isolates on test plants grown in soil. Ten downy brome (B. tectorum) seeds were seeded into 6.4 diameter by 7.6 cm deep plastic pots filled with Ritzville silt loam plus 20% sand (w/w), and four winter wheat seeds were seeded into 7.6 cm diameter by 15.2 cm deep plastic pots filled with Ritzville silt loam with 20% fine sand (w/w). Approximately 10⁸ CFU of inhibitory pseudomonads NRRL B-18293, NRRL B-18294, or NRRL B-18295 were dripped on the soil surface on the seeded area in the downy brome and winter wheat seeded pots. Controls received the fresh medium without cells. Four replicates of each treatment were included. The soil was wetted to -1/3 bar water potential, and the pots incubated in the growth chamber with a 14-hour day at 18^{o}C and a 10-hour night at 13^{o}C.

Seedling emergence was determined. After 2 weeks the seedlings were pulled up and the roots washed with water until free of soil. The roots and shoots were excised, dried at 60^{o}C for 48 hours, and dry shoot and root weight recorded. In order for the bacterial strain to be considered to be inhibitory to downy brome without deleteriously affecting wheat, the downy brome seedlings treated with the bacteria must have averaged at least 25% reduced root growth (dry root weight) when compared to control downy brome seedlings, and the winter wheat seedlings treated with the bacteria must have averaged less than 10% reduced root growth (dry root weight) when compared to control wheat seedlings. The test results for the strains are given in Table 2. As can be seen from the data, the strains passed the growth chamber test.

**TABLE 2**

| Isolate | Downy Brome | | Winter Wheat | |
|---|---|---|---|---|
| | Root wt/pot (mg) | Reduction In Root Growth Compared to Control (%) | Root wt/plant (mg) | Reduction In Root Growth Compared to Control (%) |
| Control | 37.5 | -- | 29.0 | -- |
| NRRL B-18293 | 24.0 | 36 | 40.3 | None |
| NRRL B-18294 | 27.3 | 27 | 41.3 | None |
| NRRL B-18295 | 17.0 | 55 | 31.0 | None |

### EXAMPLE 4

### Field Screening of Bacteria to Inhibit Downy Brome Growth

Strains NRRL B-18293 and NRRL B-18294 that had passed the growth chamber test were field tested in Pendleton, Oregon during the 1987-1988 growing season as follows: PMS broth was inoculated with glycerol-preserved-strains and incubated at 20^{o}C for 2 days. Agar plates of PMS medium were prepared and each inoculated with 1 ml of the PMS broth culture medium containing the individual strains. One ml of inoculum was spread on the plate with a sterile glass rod. A sufficient number of plates were inoculated to provide organisms to seed the field plots at 10⁹ CFU of the test strain/m². The plates were incubated at 15^{o}C for 3 days to obtain maximum growth. The plates were flooded with sterile deionized water, and the organisms mixed in the water by agitation of the surface of the plate with a glass rod. The plate washings for each strain were pooled. The suspension was diluted with sterile deionized water to an optical density of 0.9 (determined on a spectrophotometer at 600 nm). The amount of this solution necessary to treat the plot area was added to a sprinkling can (about 7 liter capacity) and water added to fill the can to facilitate even spreading of the organisms on the plot. For example, for a plot of 1 m², the amount of the solution necessary to contain about 10⁹ CFU of the test strain/m² was added to the sprinkling can.

The field had naturally occurring residue on it. The plots were fumigated with methyl bromide prior to planting to remove ambient weed seeds. Plots were 1 meter² with 1 meter between plots were arranged in a randomized complete block design. Each treatment was replicated four times (sprinkled with the test strain or a water-check). Before the organisms were added, the plots were hand seeded in rows onto the soil surface with downy brome (B. tectorum) with 10 seeds planted per 25 cm. Then the organisms or water treatments were sprinkled on.

Another set of identical plots as described above were established with the only difference being the mode of application of the bacteria being tested. Inoculum of each test strain was prepared as described previously incubating in PMS broth. Chopped straw, about 5 cm length, was placed in a plastic container and wetted to about -1/3 bar water potential. Then 1 ml of the PMS inoculum of each test strain or of sterile PMS broth per 100 g of straw was added (each to a separate container of straw) and mixed. Extreme care was taken to avoid contamination from one vessel to the other. The mixtures were incubated at ambient temperatures usually ranging from 22^{o}C during the day to about 12^{o}C during the night. After 5 days, counts of the applied microorganism were greater than about 10⁹ CFU per gram of straw dry weight. This was the amount desired to treat a m² of plot. The quantity of straw is too small to readily spread evenly over the plots so it was mixed with 49 g of fresh straw to facilitate spreading, and the straw plus organisms were added at a rate to give 10⁹ CFU per m² of plot. The organisms plus straw or the straw control was applied to an identical set of plots in the same configuration as described above for organisms in water or water check treatments.

In early April, a 0.3 m of the middle row from each downy brome plot was carefully dug so no roots were lost. The roots were carefully washed under flowing tap water to remove all visible soil. Tops and roots from the downy brome were excised and dried at 60^{o}C for 48 hours. In order for the bacterial strain to be considered inhibitory to downy brome in the field test, the downy brome plants treated with the bacteria must average at least 10% reduced stand, at least 10% reduced root growth (root dry weight), or at least 10% reduced shoot growth (shoot dry weight) when compared to the control downy brome plants. Table 3 shows the inhibition of downy brome by strains NRRL B-18293 and NRRL B-18294, initially determined as non-fluorescent, sprayed on the residue and soil surface (Spray) or incubated with straw before applying to the soil surface (Straw). As can be seen from the data, the strains passed the field test.

Winter wheat was grown in field plots and treated with strains NRRL B-18293 and NRRL B-18294 at 10⁹ CFU per meter² showed no difference in stand count from control winter wheat, thereby showing that neither of the test strains deleteriously affected winter wheat.

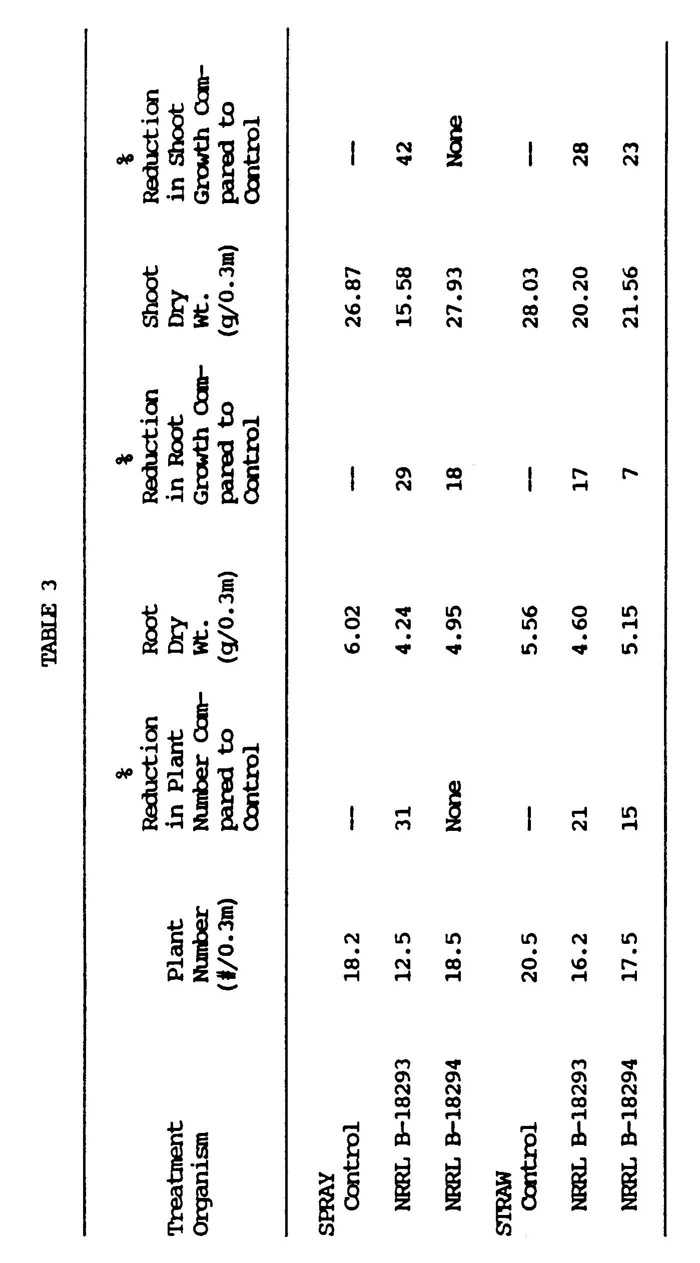

In another field test NRRL B-18295 was tested as described above for the other strains. Downy brome plants treated with this strain averaged reduced stand and reduced shoot growth (shoot dry weight) of 35% and 45%, respectively, compared to control downy brome plants. Winter wheat treated with this strain showed no reduction in stand or shoot growth compared to control winter wheat.

It is understood that the foregoing detailed description is given merely by way of illustration and that modification and variations may be made therein without departing from the spirit and scope of the invention.

## Claims

1. A method for screening bacteria to select strains which will inhibit downy brome growth in field-grown, small grain crops without deleteriously affecting the small grain crop, which comprises:
(a) isolating a strain of bacteria from the rhizoplane, rhizosphere, or both rhizoplane and rhizosphere of downy brome or a small grain crop grown in the field;
(b) screening said strain isolated in step (a) for inhibition of downy brome growth without deleteriously affecting the small grain crop in vitro as follows;
(1) growing downy brome in vitro in the presence of a cell culture, substantially cell-free culture supernatant, or cell-free culture filtrate of the strain isolated in step (a);
(2) growing downy brome as in step (b)(1) without the addition of said strain, supernatant or filtrate;
(3) selecting a strain which caused downy brome of step (b)(1) to average at least a 50% reduction in root growth or at least a 20% reduction in germination compared to downy brome grown in step (b)(2);
(4) growing the small grain crop of the variety to be protected in vitro in the presence of a cell culture, substantially cell-free culture supernatant, or cell-free culture supernatant of said strain selected in step (b)(3);
(5) growing said small grain crop of the variety to be protected as in step (b)(4) without the addition of said strain, supernatant or filtrate; and
(6) selecting a strain which caused said small grain crop of step (b)(4) to average less than 10% reduction in root growth compared to said small grain crop grown in step (b)(5);
(c) screening said strain selected in step (b)(6) for inhibition of downy brome without deleteriously affecting the small grain crop in a growth chamber as follows:
(1) Separately growing downy brome and the small grain crop of the variety to be protected in the growth chamber in the presence of said strain selected in step (b)(6) in a concentration of about 10⁸ to 10¹⁰ CFU of said strain per 10 downy brome seeds and in a concentration of about 10⁸ to 10¹⁰ CFU of said strain per 4 small grain crop seeds;
(2) growing downy brome and said small grain crop as in step (c)(1) without the addition of said strain; and
(3) selecting a strain which caused downy brome of step (c)(1) to average at least a 25% reduction in root and/or shoot growth compared to downy brome grown in step (c)(2) and which caused said small grain crop grown in step (c)(1) to average less than 10% reduction in root growth or shoot growth compared to said small grain crop grown in step (c)(2);
(d) screening said strain selected in step (c)(3) for inhibition of downy brome without deleteriously affecting the small grain crop in the field as follows:
(1) separately growing downy brome and the small grain crop of the variety to be protected in the field in the presence of said strain selected in step (c)(3) in a concentration of about 10⁷ to 10¹² CFU of said strain per meter² of said downy brome and said small grain crop;
(2) growing downy brome and said small grain crop as in step (d)(1) without the addition of said strain; and
(3) selecting a strain which caused downy brome of step (d)(1) to average at least a 10% reduction in stand, root growth, or shoot growth compared to downy brome grown in step (d)(2) and which caused said small grain crop grown in step (d)(1) to average less than 10% reduction in stand, root growth, or shoot growth compared to said small grain crop grown in step (d)(2).

2. A method for inhibiting downy brome growth comprising applying the Strain of bacteria selected in step (d)(3) according to claim 1 to field grown small grain crops of the variety to be protected in an amount sufficient to inhibit the growth of downy brome in said crop.

3. A method according to claim 2 wherein said strain is applied in a concentration of about 10⁷ to 10¹² CFU per meter².

4. A method according to claim 2 or 3 wherein said strain is applied as a spray treatment comprising said strain in a liquid carrier and/or is applied as a straw treatment comprising said strain grown on straw.

5. A method of any preceding claim, wherein said downy brome is Bromus tectorum and/or said small grain crop is winter wheat.

6. A method of inhibiting the growth of downy brome in small grain crops grown in the field, which comprises growing the small grain crop of the variety to be protected in the presence of an inhibitory amount, as determined by passing the screen test of claim 1, of a culture of bacteria which inhibit downy brome in said small grain crop under field conditions.

7. A method according to any of claims 2 to 6, wherein the selected culture of said bacteria comprises a strain of Pseudomonas.

8. A method according to claim 7 wherein said strain of Pseudomonas is selected from the group consisting of NRRL B-18293, NRRL B-18294, and NRRL B-18295.

9. A strain of Pseudomonas selected from NRRL B-18293, NRRL B-18294 and NRRL B-18295.

10. A culture of bacteria which inhibits downy brome in small grain crops under field conditions as determined by passing the screen test of claim 1.

11. A method of screening bacteria to select strains capable of inhibiting downy brome growth without substantially deleteriously affecting the growth of field-grown, small grain crops, comprising testing bacteria, preferably from the rhizoplane, rhizosphere, or both rhizoplane and rhizosphere of downy brome or of a small grain crop, under laboratory conditions, for significant inhibition of downy brome growth but without a substantial deleterious affect on the growth of the desired small grain crop.

12. A composition for inhibiting growth of downy brome in small grain crops, comprising a suitable amount of bacteria selected in accordance with the method of claim 1 or 11, together with an agronomically acceptable carrier therefor.

13. A composition according to claim 12, for use in accordance with any of claims 2 to 8.

## Patentansprüche

1. Screeningverfahren für Bakterien zur Auswahl von Stämmen, die das Wachstum der Dachtrespe in auf dem Feld wachsenden Kleinkorn-Feldfrüchten hemmen, ohne die Kleinkorn-Feldfrucht schädlich zu beeinflussen, enthaltend:
(a) Isolieren eines Bakterienstammes von der Wurzeloberfläche, der Rhizosphäre oder sowohl von der Wurzeloberfläche als auch der Rhizosphäre der Dachtrespe oder der auf dem Feld wachsenden Kleinkorn-Feldfrucht;
(b) Screening des in Stufe (a) isolierten Stamms zur Hemmung des Dachtrespenwachstums, ohne die Kleinkorn-Feldfrucht in vitro schädlich zu beeinflussen, wie folgt;
(1) Ziehen der Dachtrespe in vitro in Gegenwart einer Zellkultur, eines im wesentlichen zellfreien Kulturüberstands oder eines zellfreien Kulturfiltrats des in Stufe (a) isolierten Stamms;
(2) Ziehen der Dachtrespe wie in Stufe (b) (1) ohne Zugabe des Stamms, des Überstands oder des Filtrats;
(3) Auswahl eines Stammes, der die Dachtrespe aus Stufe (b) (1) wenigstens zu einer mittleren Verringerung des Wurzelwachstums um 50 % oder wenigstens eine Verringerung der Reimung um 20 %, verglichen mit der in Stufe (b) (2) gezogenen Dachtrespe, veranlaßt;
(4) Ziehen der Kleinkorn-Feldfrucht der in vitro zu schützenden Sorte in Gegenwart einer Zellkultur, eines im wesentlichen zellfreien Kulturüberstands oder eines zellfreien Kulturüberstands des in Stufe (b) (3) ausgewählten Stamms;
(5) Ziehen der Kleinkorn-Feldfrucht der zu schützenden Sorte wie in Stufe (b) (4) ohne Zugabe des Stamms, des Überstands oder des Filtrats und
(6) Auswahl eines Stamms, der diese Kleinkorn-Feldfrucht von Stufe (b) (4) zu einer mittleren Verringerung des Wurzelwachstums von weniger als 10 %, verglichen mit der in Stufe (b) (5) gezogenen Kleinkorn-Feldfrucht, veranlaßt;
(c) Screening des in Stufe (b) (6) ausgewählten Stamms zur Hemmung der Dachtrespe ohne schädliche Beeinflussung der Kleinkorn-Feldfrucht in einer Wachstumskammer wie folgt:
(1) Getrenntes Ziehen der Dachtrespe und der Kleinkorn-Feldfrucht der zu schützenden Sorte in der Wachstumskammer in Gegenwart des in Stufe (b) (6) ausgewählten Stamms in einer Konzentration von etwa 10⁸ bis 10¹⁰ CFU dieses Stamms auf je 10 Dachtrespensamen und in einer Konzentration von etwa 10⁸ bis 10¹⁰ CFU dieses Stamms auf je vier Samen der Kleinkorn-Feldfrucht;
(2) Ziehen der Dachtrespe und der Kleinkorn-Feldfrucht wie in Stufe (c) (1) ohne Zugabe des Stamms und
(3) Auswahl eines Stamms, der eine mittlere Verringerung von wenigstens 25 % im Wurzel- und/oder Triebwachstum bei der Dachtrespe, verglichen mit dem Dachtrespenwachstum in Stufe (c) (2), verursacht und der im Mittel eine Verringerung von weniger als 10 % im Wurzelwachstum oder Triebwachstum der in Stufe (c) (1) gezogenen Kleinkorn-Feldfrucht, verglichen mit der Kleinkorn-Feldfrucht der Stufe (c) (2), verursacht;
(d) Screening des in Stufe (c) (3) ausgewählten Stamms zur Hemmung der Dachtrespe, ohne die Kleinkorn-Feldfrucht schädlich zu beeinflussen, wie folgt:
(1) Getrenntes Ziehen von Dachtrespe und Kleinkorn-Feldfrucht der auf dem Feld zu schützenden Sorte in Gegenwart des in Stufe (c) (3) ausgewählten Stamms in einer Konzentration von etwa 10⁷ bis 10¹² CFU des Stamms je Quadratmeter Dachtrespe und Kleinkorn-Feldfrucht;
(2) Ziehen von Dachtrespe und Kleinkorn-Feldfrucht wie in Stufe (d) (1) ohne Zugabe des Stamms und
(3) Auswahl des Stamms, der die Dachtrespe aus Stufe (d) (1) zu einer mittleren, wenigstens 10 %igen Verringerung des Bestands, des Wurzelwachstums oder des Triebwachtums, verglichen mit dem Dachtrespenwachstum in Stufe (d) (2), veranlaßt und der das Wachstum der Kleinkorn-Feldfrucht in Stufe (d) (1) zu einer mittleren Verringerung von weniger als 10 % im Bestand, Wurzelwachstum oder Triebwachstum, verglichen mit dem Wachstum der Kleinkorn-Feldfrucht in Stufe (d) (2), veranlaßt.

2. Verfahren zur Hemmung des Dachtrespenwachstums, enthaltend das Aufbringen des in Stufe (d) (3) nach Anspruch 1 ausgewählten Bakterienstamms auf die im Feld gezogenen Kleinkorn-Feldfrüchte der zu schützenden Sorte in einer Menge, die ausreichend ist, das Dachtrespenwachstum in der Frucht zu hemmen.

3. Verfahren nach Anspruch 2, worin dieser Stamm in einer Konzentration von etwa 10⁷ bis 10¹² CFU je Quadratmeter ausgebracht wird.

4. Verfahren nach Anspruch 2 oder 3, worin der Stamm mittels Sprühbehandlung, den Stamm in einem flüssigen Träger enthaltend, ausgebracht wird und/oder als Strohbehandung, enthaltend den auf Stroh gezogenen Stamm, ausgebracht wird.

5. Verfahren nach einem der vorhergehenden Ansprüche, worin die Dachtrespe Bromus tectorum und/oder die Kleinkorn-Feldfrucht Winterweizen ist.

6. Verfahren zur Hemmung des Dachtrespenwachstums in auf dem Feld wachsenden Kleinkorn-Feldfrüchten, enthaltend das Ziehen der Kleinkorn-Feldfrucht der zu schützenden Sorte in Gegenwart einer hemmenden Menge, bestimmt mittels Durchführung des Screentests nach Anspruch 1, einer Bakterienkultur, welche die Dachtrespe in der Kleinkorn-Feldfrucht unter Feldbedingungen hemmt.

7. Verfahren nach einem der Ansprüche 2 bis 6, worin die ausgewählte Bakterienkultur einen Pseudomonas-Stamm enthält.

8. Verfahren nach Anspruch 7, worin der Pseudomonas-Stamm ausgewählt ist aus der aus NRRL B-18293, NRRL B-18294 und NRRL B-18295 bestehenden Gruppe.

9. Pseudomonas-Stamm, ausgewählt aus NRRL B-18293, NRRL B-18294 und NRRL B-18295.

10. Bakterienkultur, die das Dachtrespenwachstum in Kleinkorn-Feldfrüchten unter Feldbedingungen hemmt, die mittels Durchführung des Screentests nach Anspruch 1 bestimmt ist.

11. Screeningverfahren für Bakterien zur Auswahl von zur Hemmung des Dachtrespenwachstums geeigneten Stämmen ohne im wesentlichen schädliche Beeinflussung des Wachstums von auf dem Feld wachsenden Kleinkorn-Feldfrüchten, enthaltend das Prüfen von Bakterien, vorzugsweise aus der Wurzeloberfläche, der Rhizosphäre oder der Wurzeloberfläche und der Rhizosphäre der Dachtrespe oder einer Kleinkorn-Feldfrucht unter Laborbedingungen zur deutlichen Hemmung des Dachtrespenwachstums, jedoch ohne wesentliche schädliche Beeinflussung des Wachstums der gewünschten Kleinkorn-Feldfrucht.

12. Zubereitung zur Hemmung des Dachtrespenwachtums in Kleinkorn-Feldfrüchten, enthaltend eine geeignete Menge von Bakterien, ausgewählt in Übereinstimmung mit dem verfahren nach Anspruch 1 oder 11 gemeinsam mit einem landwirtschaftlich verträglichen Träger dafür.

13. Zubereitung nach Anspruch 12 zur Verwendung in Übereinstimmung mit einem der Ansprüche 2 bis 8.

## Revendications

1. Procédé de criblage de bactéries pour sélectionner des souches qui inhiberont la croissance de la brome des toits en champ parmi les céréales à petits grains cultivées en champ sans effets nocifs sur la céréale à petits grains, qui comprend :
(a) l'isolement d'une souche de bactérie à partir du rhizoplan, de la rhizosphère ou du rhizoplan et de la rhizosphère ensemble de la brome des toits ou de la céréale à petits grains cultivée en champ ;
(b) le criblage desdites souches isolées dans l'étape (a) pour l'inhibition de la brome des toits sans effets nocifs sur la céréale à petits grains in vitro comme suit ;
(1) culture de la brome des toits in vitro en présence d'une culture cellulaire, d'un surnageant de culture pratiquement dépourvu de cellules, ou d'un filtrat de culture dépourvu de cellules de la souche isolée dans l'étape (a) ;
(2) culture de brome des toits comme dans l'étape (b)(1) sans l'addition desdits souche, surnageant ou filtrat ;
(3) sélection d'une souche qui provoque chez la brome des toits de l'étape (b)(1) une réduction moyenne d'au moins 50 % de la croissance des racines ou une réduction d'au moins 20 % de la germination par rapport à la brome des toits cultivée dans l'étape (b)(2) ;
(4) culture de la céréale à petits grains de la variété à protéger in vitro en présence d'une culture cellulaire, d'un surnageant de culture pratiquement dépourvu de cellules, ou d'un filtrat de culture dépourvu de ladite souche sélectionnée dans l'étape (b)(3) ;
(5) culture de ladite céréale à petits grains de la variété à protéger comme dans l'étape (b)(4) sans l'addition desdits souche, surnageant ou filtrat; et
(6) sélection d'une souche qui provoque chez ladite céréale à petits grains de l'étape (b)(4) une réduction moyenne de moins de 10 % de la croissance des racines par rapport à ladite céréale à petits grains cultivée dans l'étape (b)(5) ;
(c) le criblage de ladite souche sélectionnée dans l'étape (b)(6) pour l'inhibition de la brome des toits sans effets nocifs sur la céréale à petits grains dans une chambre de culture comme suit :
(1) culture séparée de brome des toits et de céréale à petits grains de la variété à protéger dans la chambre de culture en présence de ladite souche sélectionnée dans l'étape (b)(6) à une concentration d'environ 10⁸ à 10¹⁰ CFU de ladite souche pour 10 graines de brome des toits et à une concentration d'environ 10⁸ à 10¹⁰ CFU de ladite souche pour 4 graines de céréale à petits grains ;
(2) culture de la brome des toits et de ladite céréale à petits grains comme dans l'étape (c)(1) sans l'addition de ladite souche ; et
(3) sélection d'une souche qui provoque chez la brome des toits de l'étape (c)(1) une réduction moyenne d'au moins 25 % de la croissance des racines et/ou des pousses par rapport à la brome des toits cultivée dans l'étape (c)(2) et qui provoque chez ladite céréale à petits grains de l'étape (c)(1) une réduction moyenne de moins de 10 % de la croissance des racines ou de la croissance des pousses par rapport à ladite céréale à petits grains cultivée dans l'étape (c)(2) ;
(d) le criblage de ladite souche sélectionnée dans l'étape (c)(3) pour l'inhibition de la brome des toits sans effets nocifs sur la céréale à petits grains en champ comme suit :
(1) culture séparée de brome des toits et de la céréale à petits grains de la variété à protéger en champ en présence de ladite souche sélectionnée dans l'étape (c)(3) à une concentration d'environ 10⁷ à 10¹² CFU de ladite souche par m² de ladite brome des toits et de ladite céréale à petits grains ;
(2) culture de la brome des toits et de ladite céréale à petits grains comme dans l'étape (d)(1) sans l'addition de ladite souche ; et
(3) sélection d'une souche qui provoque chez la brome des toits de l'étape (d)(1) une réduction moyenne d'au moins 10 % de la récolte sur pied, de la croissance des racines ou de la croissance des pousses par rapport à la brome des toits cultivée dans l'étape (d)(2) et qui provoque chez ladite céréale à petits grains de l'étape (d)(1) une réduction moyenne de moins de 10 % de la récolte sur pied, de la croissance des racines ou de la croissance des pousses par rapport à ladite céréale à petits grains cultivée dans l'étape (d)(2).

2. Procédé d'inhibition de la croissance de la brome des toits comprenant l'application de la souche de bactérie sélectionnée dans l'étape (d)(3) selon la revendication 1 sur des céréales à petits grains cultivées en champ de la variété à protéger en une quantité suffisante pour inhiber la croissance de la brome des toits parmi ladite céréale.

3. Procédé selon la revendication 2, dans lequel ladite souche est appliquée en une concentration d'environ 10⁷ à 10¹² CFU par m².

4. Procédé selon la revendication 2 ou 3, dans lequel ladite souche est appliquée par un traitement par pulvérisation comprenant ladite souche dans un véhicule liquide et/ou est appliqué par un traitement utilisant de la paille comprenant ladite souche cultivée sur de la paille

5. Procédé selon l'une quelconque des revendications précédentes, dans lequel ladite brome des toits est Bromus tectorum et/ou ladite céréale à petits grains est du blé d'hiver.

6. Procédé pour inhiber la croissance de la brome des toits parmi des céréales à petits grains cultivées en champ, qui comprend la culture de la céréale à petits grains de la variété à protéger en présence d'une quantité inhibitrice, telle que déterminée en passant le test de criblage de la revendication 1, d'une culture de bactéries qui inhibe la brome des toits parmi la céréale à petits grains en conditions de champ.

7. Procédé selon l'une quelconque des revendications 2 à 6, dans lequel la culture sélectionnée desdites bactéries comprend une souche de Pseudomonas.

8. Procédé selon la revendication 7, dans lequel ladite souche de Pseudomonas est choisie dans le groupe constitué de NRRL B-18293. NRRL B-18294, et NRRL B-18295.

9. Souche de Pseudomonas choisie parmi NRRL B-18293, NRRL B-18294, et NRRL B-18295.

10. Culture de bactérie qui inhibe la brome des toits parmi les céréales à petits grains en conditions de champ comme déterminé en passant le test de criblage de la revendication 1.

11. Procédé de criblage de bactéries destiné à sélectionner des souches capables d'inhiber la brome des toits sans effets nocifs substantiels sur la croissance de céréales à petits grains cultivées en champ, comprenant le test de bactéries, de préférence provenant du rhizoplan, de la rhizosphère ou du rhizoplan et de la rhizosphère ensemble, de brome des toits ou d'une céréale à petits grains, en conditions de laboratoire, pour l'inhibition significative de la croissance de la brome des toits sans effets nocifs substantiels sur la croissance de la céréale à petits grains désirée.

12. Composition destinée à inhiber la croissance de la brome des toits parmi les céréales à petits grains, comprenant une quantité convenable de bactéries sélectionnées selon le procédé de la revendication 1 ou 11, avec un véhicule acceptable du point de vue agronomique de celle-ci.

13. Composition selon la revendication 12, pour utilisation selon l'une quelconque des revendications 2 à 8.
